# DEMANDE DE BREVET EUROPEEN

(11) **EP 0 534 829 A1**
(43) Date de publication de la demande: **31.03.1993**
(21) Numéro de dépôt: 92402574.5
(22) Date de dépôt: 18.09.1992
(51) Int. Cl.: C23G 5/028, C07C 17/42

(54) **Procédé et additif pour la stabilisation d'une composition à base de solvants halogénés, compositons ainsi obtenues et leur application au dégraissage ou au nettoyage de pièces métalliques**

(30) Priorité: 27.09.1991 FR 9111907
(71) Demandeur: ELF ATOCHEM S.A., F-92800 Puteaux (FR)
(72) Inventeur: Michaud, Pascal, F-95210 St Gratien (FR); Martin, Jean-Jacques, F-92270 Bois-Colombes (FR)
(74) Mandataire: Hirsch, Marc-Roger

(57) **Abrégé**

Procédé de stabilisation de solvants halogénés consistant en l'ajout auxdits solvants de 0,0001 à 16,5% en poids desdits solvants, d'époxyde de limonène et composition ainsi obtenue.

## Description

La présente invention concerne un procédé de stabilisation de compositions à base de solvants halogénés; elle se rapporte en outre aux additifs employés à cette fin et aux compositions ainsi obtenues contenant de tels additifs ainsi qu'à l'application de ces compositions au dégraissage ou au nettoyage de pièces métalliques. Elle concerne également un procédé d'obtention de compositions stabilisées de solvants halogénés de formule CₙH_{z}FₚCl_{y}, dans laquelle 1 ≦ n ≦ 6, p ≧ 0, y ≧ 1, z ≧ 0, ainsi que les additifs employés à cette fin, les compositions ainsi obtenues contenant de tels additifs et l'application précitée de ces compositions.

Les solvants halogénés, par exemple, le chlorure de méthylène, le trichloréthylène, le perchloréthylène, le 1,1,1-trichloréthane , sont largement utilisés dans l'industrie. Le 1,1,1-trichloréthane (ou méthyl-chloroforme) en particulier, est utilisé pour l'obtention d'aérosols, pour le nettoyage à sec des vêtements, pour le dégraissage et/ou le nettoyage de pièces ou organes métalliques, par exemple mouillés par des huiles de coupe. Le problème posé par l'emploi de solvants organo-chlorés et/ou organo-fluorés, et en particulier le 1,1,1-trichloréthane, est leur tendance à se décomposer en présence de métaux légers ou de leurs alliages. Ce problème particulier limite donc leur emploi en tant qu'agent de dégraissage et/ou de nettoyage de pièces métalliques à base d'aluminium, de magnésium ou de leurs alliages. Il est connu que le 1,1,1-trichloréthane, en présence d'aluminium frotté ou gratté, est instable et se décompose, en une période de temps relativement courte, en formant une boue goudronneuse noire dont la composition est pratiquement incontrôlable. Cette décomposition s'accompagne d'une modification du pH de la solution qui, en fonction de l'huile de coupe utilisée, devient basique ou acide, avec dans ce dernier cas une formation d'acide chlorhydrique. De plus, lorsqu'un solvant organo-chloré et/ou organo-fluoré, sous forme de film mince, est soumis à une pression entre deux feuilles d'aluminium, la tendance très nette à la décomposition de ce solvant est aggravée. La décomposition de ces solvants a aussi lieu en phase vapeur, lors des opérations de dégraissage à chaud. De nombreux additifs ont donc été proposés afin d'empêcher, ou au moins de minimiser, la décomposition de ces solvants, conduisant à leur coloration, à la formation de sous-produits indésirables et à la perte de leurs propriétés solvantes.

Les divers additifs proposés afin de stabiliser les solvants halogénés peuvent être des nitro-alcanes, des alcools saturés et insaturés, des éthers, des amines, des oxétanes, des hétérocycles azotés, des nitriles, des esters, des dérivés phénoliques, des dérivés époxys, et leurs mélanges. Les dérivés époxys sont couramment utilisés, seuls ou en mélange avec les autres stabilisants connus dans l'art. L'OBu (oxyde de butylène) est actuellement le stabilisant le plus couramment utilisé, en raison de son prix. Plusieurs composés ont été proposés afin de remplacer l'OBu, dont des époxys particuliers.

EP-A-044 111 (Solvay) décrit des compositions stabilisées de solvants halogénés contenant du 2-méthyl-2,3-époxy-butane. Wacker Chemie propose plusieurs époxys susceptibles de remplacer l'OBu, dont par exemple: cyclohexène oxyde, 4-vinyl-cyclohexène, 7,8-époxy-octène. Classiquement, les époxys utilisés sont choisis parmi: l'époxypropane, l'époxybutane, les 2-méthyl-époxypropane, 2-méthylépoxybutanes et l'épichlorhydrine.

Aucune publication n'enseigne, ni ne suggère, l'emploi particulier de l'époxy selon l'invention, ni les effets surprenants et inattendus auxquels il conduit.

La présente invention a pour objet un additif pour la stabilisation d'une composition de solvants halogénés de formule CₙH_{z}FₚCl_{y}, dans laquelle 1 ≦ n ≦ 6, p ≧ 0, y ≧ 1, z ≧ 0, contenant de 0,0001% à 16,5% en poids, d'époxyde de limonène.

Le terme "époxyde de limonène" tel qu'utilisé dans la présente invention signifie l'époxyde 1,2 de limonène, c'est à dire que la fonction époxy est portée par la double liaison située sur le cycle.

Selon une forme d'exécution de la présente invention, l'additif comprend de plus au moins un des co-stabilisants suivants:
a) époxy en C₂ à C₇ différent du d'époxyde de limonène,
b) alcool saturé en C₁ à C₇,
c) alcool insaturé en C₃ à C₆,
d) nitro-alcane en C₁ à C₄,
e) éther interne mono- ou poly-cyclique,
f) amine aliphatique en C₃ à C₁₀ ou aromatique,
g) hétérocycle contenant de l'azote.

Selon une autre forme d'exécution de la présente invention, les composants a), b), c), d), e), f) et g) représentent respectivement de:
a) 0% à 16,50%
b) 0,001 à 16,50%
c) 0,001 à 16,50%
d) 0,001 à 10,00%
e) 0,001 à 16,50%
f) 0 à 3,33%
g) 0 à 3,33%
exprimés en poids par rapport au poids dudit additif.

La présente invention a également pour objet un procédé de stabilisation d'une composition stabilisée de solvants halogénés de formule CₙH_{z}FₚCl_{y}, dans laquelle 1 ≦ n ≦ 6, p ≧ 0, y ≧ 1, z ≧ 0, consistant à ajouter auxdits solvants de 0,01% à 5% en poids, par rapport au poids de la composition, d'époxyde de limonène. De préférence, ce procédé consiste à ajouter de 0,2% à 3% en poids, par rapport au poids de la composition, d'époxyde de limonène.

Selon un mode de réalisation de la présente invention, la composition comprend de plus au moins un des co-stabilisants suivants:
a) époxy en C₂ à C₇ différent de l'époxyde de limonène,
b) alcool saturé en C₁ à C₇,
c) alcool insaturé en C₃ à C₆,
d) nitro-alcane en C₁ à C₄
e) éther interne mono- ou poly-cyclique,
f) amine aliphatique en C₃ à C₁₀ ou aromatique,
g) hétérocycle contenant de l'azote.

L'époxy en C₂ à C₇ différent de l'époxyde de limonène représente en poids, par rapport au poids de la composition, de 0 à 5%, de préférence de 0 à 3%, et et choisi parmi: l'époxypropane, l'époxybutane, les 2-méthyl-époxypropane, 2-méthyl-époxybutanes, l'épichlorhydrine et leurs mélanges.

Les alcools saturés en C₁ à C₇ représentent en poids, par rapport au poids de la composition, de 0,1 à 5%, de préférence de 0,5 à 3,5%. Des exemples d'alcools saturés sont le méthanol, l'éthanol, le propanol, l'isopropanol, le butanol-1, le butanol-2, l'alcool butylique tertiaire, les pentanols tels que l'alcool amylique tertiaire, le méthylcellosolve, l'éthylcellosolve, le propylcellosolve, le butylcellosolve, la glycérine, l'éthylèneglycol, le propylèneglycol, la 2-hydroxy-2-méthyl-3-butanone, le 3,3-diméthoxy-2-méthylbutanol-2 et le 3-amino-2-méthylbutanol-2. Les alcools saturés préférés sont: les propanols, les butanols tertiaire et secondaire et le pentanol tertiaire. L'alcool saturé particulièrement préféré est le butanol tertiaire.

Les alcools insaturés en C₃ à C₆ représentent en poids, par rapport au poids de la composition, de 0,1 à 5%, de préférence de 0,3 à 3%. Les alcools insaturés préférés sont: l'alcool allylique, le 2-méthyl-3-butène-2-ol et le 2-méthyl-3-butyne-2-ol. L'alcool insaturé particulièrement préféré est le 2-méthyl-3-butyne-2-ol.

Les nitro-alcanes en C₁ à C₄ représentent en poids, par rapport au poids de la composition, de 0,1 à 3%, de préférence de 0,5 à 1,5%. Les nitro-alcanes préférés sont le nitrométhane, le nitroéthane, les nitropropanes-1 ou -2 et leurs mélanges. Tout particulièrement préférés sont le nitrométhane et/ou le nitroéthane.

Les éthers internes représentent en poids, par rapport au poids de la composition, de 0,1 à 5%, de préférence de 0,5 à 2,5%. Les éthers internes appropriés pour être utilisés comme co-stabilisants comprennent les éthers mono- ou polycycliques, pouvant contenir éventuellement d'autres hétéroatomes, contenant un ou plusieurs cycles à 5 ou 6 atomes de carbone saturés ou non, substitués ou non, tels que le 1,3-dioxanne, le 1,4-dioxanne, le 1,3,5-trioxanne, le 1,3-dioxolanne, le dioxène, le dioxadiène, le thioxanne, l'oxazole, le tétrahydrofuranne, l'oxyde de diphényle, etc. L'éther préféré est le 1,3,5-trioxanne.

Les amines aliphatiques en C₃ à C₁₀ ou aromatiques représentent en poids, par rapport au poids de la composition, de 0 à 1%, de préférence de 0,005 à 0,1%. Ces amines sont celles comprenant de 3 à 10 atomes de carbone substituées ou non, telles que les amines aliphatiques secondaires et tertiaires ainsi que les amines aromatiques telles que l'aniline éventuellement substituée par des groupements alkyles en C₁ à C₄ ou des atomes de chlore. Les amines préférées sont la triéthylamine, la diisopropylamine, l'amylamine et l'hexylamine. Particulièrement préférée est la diisopropylamine.

Les hétérocycles azotés représentent en poids, par rapport au poids de la composition, de 0 à 1%, de préférence 0,005 à 0,1%. Ceux-ci sont ceux contenant de 4 à 12 atomes de carbone et de 1 à 2 atomes d'azote dans la molécule, tels que la pyridine, les picolines, la phénothiazine, la morpholine, le pyrrole et la pyrrolidine ainsi que leurs dérivés substitués par des groupements alkyles sur l'atome d'azote. Les hétérocycles préférées sont la N-méthylmorpholine, la diisopropylamine, la pyrrolidone et le N-méthylpyrrole. Les hétérocycles tout particulièrement préférés sont la N-méthylmorpholine et le N-méthylpyrrole.

Les compositions stabilisées selon la présente invention sont des compositions de solvants halogénés. Ceux-ci ont pour formule: CₙH_{z}FₚCl_{y} dans laquelle 1 ≦ n ≦ 6, p ≧ 0, y ≧ 1, z ≧ 0. Le nombre z peut être nul ou non, en fonction du degré de substitution et d'une insaturation du squelette carbonée; ainsi, z est ajusté de sorte à conserver la tétravalence du carbone. Des exemples de solvants sont le chlorure de méthylène, le trichloréthylène, perchloréthylène et le 1,1,1-trichloréthane, et leurs mélanges. Parmi ceux-ci, le solvant préféré est le 1,1,1-trichloréthane.

Les compositions stabilisées selon la présente invention peuvent en outre contenir d'autres co-stabilisants classiques, tels que des esters, des nitriles, des cétones, des composés phénoliques et des composés oléfiniques, qui peuvent représenter jusqu'à 5% en poids, par rapport au poids de la composition.

Les esters utilisables comme co-stabilisants sont des mono- et di-esters contenant de 2 à 8 atomes de carbone saturés ou non, pouvant être substitués par des halogènes ou des groupes hydroxyles tels que les formiates d'éthyle, de propyle, d'isopropyle, de butyle, de sec-butyle, de tert-butyle, d'amyle, d'hexyle et de cyclohexyle, les acétates de méthyle, d'éthyle, de propyle, d'isopropyle, de butyle, d'amyle, d'éthynyle ou de propynyle, les propionates de méthyle, d'éthyle, de propyle, d'isopropyle, de butyle, d'amyle et d'éthynyle, les butyrates de méthyle, d'éthyle, de propyle, d'isopropyle et de 2-propynyle, les acrylates et méthacrylates de méthyle, d'éthyle, de propyle, d'isopropyle, de butyle, de sec-butyle et de tert-butyle, les crotonates et isocrotonates de méthyle et d'éthyle, les sorbiates d'éthyle et de méthyle, le diformiate d'éthyle et le diacétate d'éthyle. Les monoesters non substitués contenant de 4 à 6 atomes de carbone donnent de bons résultats. De très bons résultats ont été obtenus avec l'acétate d'éthyle, l'acétate d'isobutyle, l'acétate de n-propyle, le méthacrylate de méthyle et l'acétate de n-butyle. Le méthacrylate de méthyle, l'acétate d'éthyle et l'acétate de n-propyle se sont révélés particulièrement appropriés.

Les nitriles utilisables comme co-stabilisants sont des composés aliphatiques ou aromatiques, saturés ou non saturés, éventuellement substitués, contenant de 2 à 7 atomes de carbone tels que l'acétonitrile, le propionitrile, l'acrylonitrile, le butyronitrile, le méthacrylonitrile, le malonitrile, le benzonitrile, le diméthylaminoacétonitrile, le méthylaminopropionitrile, le diméthylaminopropionitrile, le diéthylaminoacétonitrile, le méthyléthylaminoacétonitrile, le thiodipropionitrile, le tétracyanoéthylène, le tétracyanoquinodiméthane, le tétracyanodithiine, le 1,2-dicyano-1,2-bis (trifluorométhyl)éthylène, la cyanobenzaldéhyde, le nitrobenzonitrile, la cyanopyridine. Parmi ceux-ci, l'acétonitrile, le propionitrile et l'acrylonitrile sont les nitriles préférés.

Les cétones utilisables comme co-stabilisants sont les cétones contenant de 3 à 7 atomes de carbone substituées ou non, telles que par exemple l'acétone, la méthyléthylcétone, la diéthylcétone, la méthylpropylcétone, la méthylisopropylcétone, la 1-(2-furyl)propanone, la 3-hydroxy-2,5-hexanedione, la 2-méthyl-2-hydroxy-4-pentanone, etc.

Les composés phénoliques habituellement utilisés comme co-stabilisants peuvent comporter un ou plusieurs groupes hydroxyles liés à un noyau benzénique éventuellement déjà substitué par des groupements alkyles. Les composés préférés sont le phénol et ses dérivés alkylés tels que le thymol, les crésols, le paraméthoxyphényl, les méthyl-di-tert-butylphénols et les diméthyl-tert-butylphénols. Tout particulièrement préférés sont le phénol, le thymol et le p-méthoxyphénol.

Les composés oléfiniques utilisables comme co-stabilisants sont en général des composés comportant une ou plusieurs doubles liaisons dans la molécule et comportant de 4 à 10 atomes de carbone. Parmi ceux-ci, les composés préférés sont l'amylène tertiaire et le diisobutylène.

D'autres composés et notamment les alkoxyalcanes tels que les alkoxyéthanes, les nitrates tels que les nitrates d'alkyle contenant de 1 à 5 atomes de carbone, les quinones telles que l'hydroquinone et les anthraquinones, les hydrazines telles la diméthylhydrazine, et des composés aromatiques tels que le toluène, peuvent également être utilisés comme co-stabilisants.

Les exemples suivants sont donnés à titre illustratif de la présente invention et ne doivent en aucun cas être considérés comme limitatifs de celle-ci.

### EXEMPLE 1

On a réalisé une composition de 1,1,1-trichloréthane renfermant les stabilisants suivants:

| | % en poids |
|---|---|
| . Epoxyde de limonène | 0,91 |
| . alcool butylique tertiaire | 2,2 |
| . méthyl-2-butyne-3-ol-2 | 0,86 |
| . nitrométhane | 0,51 |
| . nitroéthane | 0,81 |
| . N-méthylmorpholine | 0,005 |

On a soumis la composition qui en résulte à l'épreuve des distillations successives qui consiste à distiller 4 fois successivement et en ne recueillant à chaque distillation que les premiers 90% en volume qui passent. Sur chacun des 4 distillats, on procède au test de grattage de plaques d'aluminium suivant la norme ASTM D 2943-71 T.

Ce test de grattage a pour objet de déterminer l'aptitude de l'association de stabilisants dans le 1,1,1-trichloréthane à éviter la dégradation de ce solvant chloré en présence d'aluminium ou d'alliages d'aluminium. Il consiste à prendre un morceau de métal nettoyé et dégraissé, qualité AA 1100 (norme ASTM correspondant à l'aluminium A 45 symbole AFNOR), à l'immerger dans 50 cm3 de 1,1,1-trichloréthane stabilisé, à température ambiante, et à le gratter à l'aide d'une tige en acier doux. Au bout d'un temps suffisant (1 heure) pour laisser se produire une réaction quelconque, on note la présence ou l'absence de bulles, la coloration du solvant ou la présence de produits résineux foncés. Le 1,1,1-trichloréthane est convenablement stabilisé et l'on n'observe aucune réaction.

La composition ci-dessus passe cette épreuve sans observer aucune trace de réaction sur les plaques d'aluminium.

Pour vérifier l'aptitude de la composition à piéger l'acide chlorhydrique, nous avons soumis la composition au test dit de l'"Acid Acceptance" selon la norme ASTM D 2942. L'"Acid Acceptance" mesuré en équivalent poids NaOH est de 0,240.

La composition est soumise à un test BAM, test dont les conditions appliquées aux solvants chlorés sont drastiques. Aucune exothermicité n'est observée, et la composition satisfait au test BAM en entier (6 batch). La composition est donc particulièrement stable.

### EXEMPLE 2

Composition à base de 1,1,1-trichloréthane contenant:

| | % en poids |
|---|---|
| . Epoxyde de limonène | 0,74 |
| . oxyde de butylène | 0,1 |
| . méthyl-2-butyne-3-ol-2 | 0,86 |
| . nitrométhane | 0,51 |
| . nitroéthane | 0,81 |
| . N-méthylmorpholine | 0,0085 |

Cette composition passe les tests décrits précédemment. L'"Acid Acceptance" mesurée est de 0,24.

### EXEMPLE 3

Composition à base de trichloréthylène contenant :

| | % en poids |
|---|---|
| . Epoxyde de limonène | 0,84 |
| . Triéthylamine | 0,0075 |
| . N-méthylpyrrole | 0,030 |
| . Acétate d'isopropyle | 0,28 |
| . Di-isobutylène | 0,28 |

Cette composition est soumise à un test à l'aluminium selon la procédure suivante: 100ml de la composition, 100ml de toluène, 18g d'aluminium et 0,7g de chlorure d'aluminium, sont introduits dans un ballon surmonté d'un réfrigérant et portés à reflux 9 heures, laissés au repos 15 heures, et à nouveau portés à reflux pour 9 heures.
Il n'est observé aucune exothermicité de réaction, la composition est donc parfaitement stable.

### EXEMPLE 4

Composition à base de 1,1,1-trichloréthane contenant :

| | % en poids |
|---|---|
| . Epoxyde de limonène | 0,91 |
| . Alcool butylique tertiaire | 2,2 |
| . Méthyl-2-butyne-3-d-2 | 0,86 |
| . 1,3-dioxolanne | 1,42 |
| . N-méthylmorpholine | 0,005 |

"L'acid acceptance" mesurée est de 0,24. Le test de grattage mis en oeuvre avec cette composition ne provoque aucune réaction. Cette composition est donc stable.

### EXEMPLE 5 (comparatif) :

La composition de l'exemple 1 est reprise à l'exception du fait que les 0,91g d'époxyde de limonène sont remplacés par 0,45g d'oxyde de butylène OBu. Les teneurs en moles sont donc substantiellement identiques, égales à 0,0064 et 0,0063 respectivement.La composition d'OBu ainsi que la composition selon l'exemple 1 sont soumises à un test d'hydrolyse à teneur molaire égale. Celui-ci est mis en oeuvre comme suit: 50ml de composition et 50ml d'eau sont introduits dans un ballon surmonté d'un réfrigérant et portés à reflux. Un prélèvement de 5ml est effectué afin de déterminer "l'acid acceptance" selon la méthode décrite dans la norme ASTM D 2942. Les résultats sont donnés dans le tableau suivant :

| TEMPS (h) | 0 | 2 | 3 | 4 |
|---|---|---|---|---|
| Composition de l'exemple 1 | 0,215 | 0,203 | 0,170 | 0,144 |
| Composition avec OBu | 0,218 | 0,138 | 0,077 | 0,014 |

Les résultats donnés dans le tableau montrent clairement que la composition à base d'époxyde de limonène est plus stable que la composition à base d'OBu.

Bien entendu, la présente invention n'est pas limitée aux modes de réalisation décrits et représentés, mais elle est susceptible de nombreuses variantes accessibles à l'homme de l'art sans que l'on ne s'écarte de l'esprit de l'invention.

## Revendications

**1.-** Additif pour la stabilisation d'une composition de solvants halogénés de formule CₙH_{z}FₚCl_{y}, dans laquelle 1 ≦ n ≦ 6, p ≧ 0, y ≧ 1,z ≧ 0, caractérisée en ce qu'il contient de 0,0001 à 16,5% en poids, d'époxyde de limonène.

**2.-** Additif selon la revendication 1, caractérisé en ce qu'il contient de plus au moins un des co-stabilisants suivants:
a) époxy en C₂ à C₇ différent de l'époxyde de limonène,
b) alcool saturé en C₁ à C₇,
c) alcool insaturé en C₃ à C₆,
d) nitro-alcane en C₁ à C₄,
e) éther interne mono- ou poly-cyclique,
f) amine aliphatique en C₃ à C₁₀ ou aromatique,
g) hétérocycle contenant de l'azote.

**3.-** Additif selon la revendication 1 ou 2, caractérisé en ce les composants a), b), c), d), e), f) et g) représentent respectivement de:
a) 0% à 16,50%
b) 0,001 à 16,50%
c) 0,001 à 16,50%
d) 0,001 à 10,00%
e) 0,001 à 16,50%
f) 0 à 3,33%
g) 0 à 3,33%
exprimés en poids par rapport au poids dudit additif.

**4.-** Procédé de stabilisation d'une composition à base de solvants halogénés de formule CₙH_{z}FₚCl_{y}, dans laquelle 1 ≦ n ≦ 6, p ≧ 0, y ≧ 1,z ≧ 0, caractérisée en ce qu'il consiste à ajouter auxdits solvants de 0,01% à 5% en poids, par rapport au poids desdits solvants, de l'époxyde de limonène.

**5.-** Procédé selon la revendication 4, caractérisé en ce qu'il consiste à ajouter de 0,2% à 3% en poids, par rapport au poids de ladite composition, d'époxyde de limonène.

**6.-** Procédé selon la revendication 4 ou 5, caractérisé en ce qu'il consiste à ajouter en outre à ladite composition au moins un des co-stabilisants suivants:
a) époxy en C₂ à C₇ différent de l'époxyde de limonène,
b) alcool saturé en C₁ à C₇,
c) alcool insaturé en C₃ à C₆,
d) nitro-alcane en C₁ à C₄,
e) éther interne mono- ou poly-cyclique,
f) amine aliphatique en C₃ à C₁₀ ou aromatique,
g) hétérocycle contenant de l'azote.

**7.-** Procédé selon la revendication 6, dans lequel les composants a), b), c), d), e), f) et g) représentent respectivement de:
a) 0% à 5%
b) 0,1 à 5%
c) 0,1 à 5%
d) 0,1 à 3%
e) 0,1 à 5%
f) 0,001 à 1%
g) 0 à 1%
exprimés en poids par rapport au poids de ladite composition.

**8.-** Procédé selon l'une quelconque des revendications 6 ou 7, dans lequel le composant a) représente de 0% à 3% en poids du poids de ladite composition et est choisi dans le groupe comprenant: époxypropane, époxybutane, 2-méthyl-époxypropane, 2-méthyl-époxybutanes, épichlorhydrine et leurs mélanges.

**9.-** Procédé selon l'une quelconque des revendications 6 à 8, dans lequel le composant b) représente de 0,5 à 3,5% en poids du poids de ladite composition et est choisi dans le groupe comprenant: méthanol, l'éthanol, le propanol, l'isopropanol, le butanol-1, le butanol-2, l'alcool butylique tertiaire, les pentanols tels que l'alcool amylique tertiaire, le méthylcellosolve, l'éthylcellosolve, le propylcellosolve, le butylcellosolve, la glycérine, l'éthylèneglycol, le propylèneglycol, la 2-hydroxy-2-méthyl-3-butanone, le 3,3-diméthoxy-2-méthylbutanol-2 et le 3-amino-2-méthylbutanol-2, et consiste, de préférence, en propanol, tert-et sec-butanol, tert-pentanol et leurs mélanges.

**10.-** Procédé selon l'une quelconque des revendications 6 à 9, dans lequel le composant c) représente de 0,3 à 3% en poids du poids de ladite composition et est choisi dans le groupe consistant en: alcool allylique, 2-méthyl-3-butène-2-ol, 2-méthyl-3-butyne-2-ol et leurs mélanges.

**11.-** Procédé selon l'une quelconque des revendications 6 à 10, dans lequel le composant d) représente de 0,5 à 1,5% en poids du poids de ladite composition et est choisi dans le groupe comprenant: nitrométhane, nitroéthane, nitropropane-1, nitropropane-2 et leurs mélanges.

**12.-** Procédé selon l'une quelconque des revendications 6 à 11, dans lequel le composant e) représente de 0,5 à 2,5% en poids du poids de ladite composition et est choisi dans le groupe comprenant les éthers mono- ou poly-cycliques, pouvant contenir éventuellement d'autres hétéroatomes, contenant un ou plusieurs cycles à 5 ou 6 atomes de carbone saturés ou non, substitués ou non, et consiste de préférence en le 1,3-dioxanne, le 1,4-dioxanne, le 1,3,5-trioxanne, le 1,3-dioxolanne, le dioxène, le dioxadiène, le thioxanne, l'oxazole, le tétrahydrofuranne, l'oxyde de diphényle.

**13.-** Procédé selon l'une quelconque des revendications 6 à 12, dans laquelle le composant f) représente de 0,005 à 0,1% en poids du poids de ladite composition et est choisi dans le groupe comprenant les amines aliphatiques secondaires et tertiaires ainsi que les amines aromatiques telles que l'aniline éventuellement substituée par des groupements alkyles en C₁ à C₄ ou des atomes de chlore, et consiste de préférence en triéthylamine, diisopropylamine, amylamine, hexylamine.

**14.-** Procédé selon l'une quelconque des revendications 6 à 13, dans lequel le composant g) représente de 0,005 à 0,1% en poids du poids de ladite composition et est choisi dans le groupe des hétérocycles contenant de 4 à 12 atomes de carbone et de 1 à 2 atomes d'azote dans la molécule, tels que la pyridine, les picolines, la phénothiazine, la morpholine, le pyrrole et la pyrrolidine ainsi que leurs dérivés substitués par des groupements alkyles sur l'atome d'azote.

**15.-** Procédé selon l'une quelconque des revendications 4 à 14, caractérisé en ce que sont ajoutés auxdits solvants d'autres co-stabilisants classiques, tels que des esters, des nitriles, des cétones, des composés phénoliques et des composés oléfiniques, qui peuvent représenter jusqu'à 5% en poids, par rapport au poids de la composition.

**16.-** Procédé selon la revendication 15, caractérisé en ce que lesdits co-stabilisants classiques sont l'acétate d'isopropyle et/ou le di-isobutylène.

**17.-** Composition stabilisée de solvants halogénés de formule CₙH_{z}FₚCl_{y}, dans laquelle 1 ≦ n ≦ 6, p ≧ 0, y ≧ 1, z ≧ 0, obtenue à l'aide du procédé selon l'une quelconque des revendications 4 à 16.

**18.-** Composition stabilisée selon la revendication 17, caractétisée en ce que le solvant est choisi dans le groupe consistant en: 1,1,1-trichloréthane, trichloréthylène, perchloréthylène, chlorure de méthylène, et leurs mélanges.

**19.-** Utilisation d'une composition stabilisée selon la revendication 17 ou 18, pour le dégraissage et/ou le nettoyage de pièces métalliques.
